# EUROPEAN PATENT APPLICATION

(11) **EP 4 570 178 A1**
(43) Date of publication of application: **18.06.2025**
(21) Application number: 23215661.2
(22) Date of filing: 11.12.2023
(51) Int. Cl.: A61B 5/16, A61B 5/00

(54) **APPARATUS, SYSTEM AND METHODS FOR IN-SITU ANNOTATION OF PHYSIOLOGICAL DATA**

(71) Applicant: University of Science, VNUHCM, Ho Chi Minh City (VN)
(72) Inventor: LE, Khanh-Duy, Ho Chi Minh City (VN); TRAN, Minh-Triet, Ho Chi Minh City (VN); NINH, Van-Tu, Whitehall, Dublin 9 (IE); LE, Tu-Khiem, Whitehall, Dublin 9 (IE); GURRIN, Cathal, Kilbarrack, Dublin 5 (IE)
(74) Representative: Cross, James Peter Archibald

(57) **Abstract**

An augmented or mixed reality apparatus comprises a display (121), one or more environmental sensors (119b) including a camera (124); a memory (1008;1010) to store video data (127) from the camera, and event data (117b) identifying at least one events (301) detected in data from one or more physiological sensors (111); and one or more processors (1004) configured to process the video data to detect one or more segments of the video data corresponding to the at least one identified events (301) and to determine label data (133) comprising emotion information associated with the at least one identified event; retrieve one or more segments of the video data corresponding to a selected one of said at least one identified event; display to a user the retrieved one or more segments of the video data corresponding to the selected event; prompt the user to input a quantitative level of emotion relating to the selected event; and include said quantitative level in the label data (133) corresponding to the selected event. The apparatus may alternatively compare physiological sensor data corresponding to the retrieved segment(s) of video data with physiological sensor data corresponding to one or more known emotional states; determine, based on said comparison, one or more candidate emotional states corresponding to the retrieved segment(s) of video data; output said one or more candidate emotional states to the user; and in response to confirmation of an emotional state by the user, annotate the event data corresponding to the retrieved segment(s) of video data with label data indicating the emotional state confirmed by the user.

## Description

### Field of the Invention

The present invention relates generally to data processing systems, and more particularly to techniques for improved labelling of captured physiological sensor data.

### Background

With the advancement and prevalence of wearable health tracking devices such as smart-watches or activity trackers, detecting a user's mental state (emotions, feelings) (e.g., stressed, angry, nervous) from his/her physiological signals (e.g., heart rate, blood pressure, blood volume, etc.) from these devices to provide timely and suitable advice and intervention has been emerging as a promising approach for healthcare and treatment. To effectively detect the user's mental state on these signals, it is crucial to collect the physiological data from the user with correct labels of the user's mental state to train detection systems. However, collecting such correctly labelled physiological data is challenging, especially in daily life settings, mainly because perceived mental states are very individual-dependent. For example, one can feel angry when his/her heart rate is lower than the normal heart rate of another person. Even though research has suggested some common physiological patterns for certain mental states of humans, there are still huge variants in the reality, making algorithms based on common datasets imprecisely detect mental states of certain individuals.

Currently researchers and developers mainly collect labelled physiological data of user mental states in lab-based settings and in post-hoc manners. In lab-based settings, physiological data of a user is collected while he/she is required to perform certain standardized tasks relevant to the mental states of interest. The user will be also required to answer some form of questionnaires to subjectively confirm their mental states while performing the task. This subjective confirmation will be then used to label the collected physiological data. However, the limitation of this approach is that the set of tasks used in the lab is typically much smaller and less diverse than the actual activities or scenarios that can trigger the mental state of interest that the user can encounter in their daily life. Also, the physiological data corresponding to the mental state of interest triggered by real-life activities and scenarios can be different from the one triggered by the lab-based task, thus potentially reducing the performance of the detection algorithm. In post-hoc data labelling, the user will be asked to review some data collected at certain periods during the day and indicate how he/she felt at that time. The reported feeling will be used as the label for the physiological data. Nevertheless, the labels collected using this approach might not be accurate as the feeling the user reported might not be original anymore when a certain time has passed.

What is desired are improved technical systems and methods to provide effective and accurate labelling of captured physiological signals.

### Summary of the Invention

Aspects of the present invention are set out in the accompanying claims.

According to at least some embodiments, there is provided an augmented or mixed reality apparatus which is arranged to identify an event based on physiological signals from a user perceiving the event. Video of the event is captured by a camera carried or worn by the user. The event is labelled with emotion information, based for example on the physiological signals captured from the user and/or the video of the event. The video of the event may be played back to the user and the user may be prompted to indicate or confirm the emotion information associated with the event.

In one embodiment, the apparatus prompts the user to indicate the quantitative level of emotion associated with the event, for example by playing back video of a previously captured event having similar emotion information and asking the user to indicate the difference in emotional levels between the two events.

In an embodiment in which the user is unable to identify an emotion associated with an event, the apparatus may present to the user similar emotional states based on similar physiological signals from other events and their corresponding labels. The apparatus may automatically generate a suggested emotion based on the similar emotional states, for example using a pre-trained AI system. The user may then confirm the suggested emotion, or input another emotion.

In some embodiments, the apparatus provides an improved user interface to intuitively perform in-situ annotation of their mental states on captured physiological data. The user may wear an AR headset that is capable of recording audio-visual data (videos and audios) of that the user is seeing and hearing and virtually displaying digital contents in front the user's eyes; and one or multiple wearable devices, such as smart watches, bands or bracelets, that are able to capture the user's physiological signals (e.g., heart rates, blood pressures, etc.). The headset and the wearable device(s) are wirelessly connected and the data captured by each device can be transmitted and synchronized among them. On each device, there is an application running to continuously capture the corresponding (audio-visual or physiological) data from the user, synchronize with the other device(s) and detect potential clues or triggers of non-neutral mental states (e.g., stressed, angry, anxious, nervous) from the captured data. Once a potential clue or trigger is detected, the system will display the corresponding audio-visual segment on the AR headset in front of the user's eyes. The displayed content will ask the user to confirm that the audio-visual content triggered a certain mental state in the user. The user can interact with this content to confirm if he was having the indicated mental state during the event shown in the audio-visual data was happening. The system receives the user input and uses the confirmation to label the corresponding captured physiological signal as well as the audio-visual data to improve the performance of the detection algorithms.

In other aspects, there are provided methods of operation of the apparatus and computer programs comprising machine readable instructions arranged to cause a programmable device to carry out the methods.

### Brief Description of the Drawings

There now follows, by way of example only, a detailed description of embodiments of the present invention, with references to the figures identified below.
Figure 1 is a block flow diagram showing the main components of a data processing system according to an embodiment of the invention.
Figure 2 is a block data-flow diagram showing an exemplary implementation of an event detector engine as shown in Figure 1.
Figure 3 schematically illustrates an exemplary record of tracked object data.
Figure 4 is a block data-flow diagram showing an exemplary implementation of an emotion recogniser engine as shown in Figure 1.
Figure 5 is a block data-flow diagram showing an exemplary implementation of an event annotator engine as shown in Figure 1.
Figure 6 is schematic illustration of an example virtual interface output to the display of the AR headset.
Figure 7 is a flow diagram illustrating the main processing steps performed by components of the AR headset of Figure 1 according to an embodiment.
Figure 8 is a flow diagram illustrating processing steps performed by a wearable device of Figure 1 according to an exemplary implementation.
Figure 9, which comprises Figure 9A to 9C, shows an example of virtual interfaces as seen through the user's AR headset.
Figure 10 is a flow diagram illustrating processing steps in an implementation of an interface allowing the user to identify the level of an emotional state relating to a candidate event in comparison with a most similar other event.
Figure 11 is an example display in the interface implemented by the process of Figure 10.
Figure 12 is a flow diagram illustrating processing steps in an implementation of an interface enabling the user to label a new emotional state.
Figure 13 is an example display in the interface implemented by the process of Figure 12, in which the physiological data relating to a candidate event is displayed in comparison with physiological data relating to previously identified emotional states.
Figure 14 is an example display in the interface implemented by the process of Figure 12, in which the system displays a suggested label for the user's emotional state in relation to the candidate event.
Figure 15 is a diagram of an example of a computing device on which one or more of the functions of the embodiments may be implemented.

### Description of Embodiments

A system consisting of an apparatus in which the user wears an augmented reality (AR) or mixed reality (MR) computing device, such as a headset or smart glasses etc., capable of capturing audio-visual signals (videos and audios) of what the user is seeing or hearing, and one or multiple wearable device(s) such as a smart-watch, bracelet or armband, that can record real-time physiological signal data of the user, such as heart rate, blood pressure, etc. Augmented or mixed reality technology allows virtual imagery to be mixed with a user's actual view of the real world. A display may be worn by the user to view the mixed imagery of virtual and real objects, in front of the user's eyes. The AR/MR computing device and wearable devices are wirelessly connected and configured to exchange captured data with each other.

On each device, there is an application module, running in the background for example, to continuously collect the corresponding data on the device, such as captured video and audio data on the AR headset and physiological signals from the connected wearable device(s). The application can also detect potential events in the captured data that might trigger certain un-neutral emotional or mental states on the user, such as stressed, angry, nervous, anxious, etc. Depending on the signal, the detection process could be performed differently.

Thereby, the present invention advantageously provides for improved processing and memory efficiencies by offloading data annotation tasks from a centralised data processing server to each user device itself. The system provides more accurate behaviour analysis, solving a technical problem of how to technically implement means for improving the mental/emotional state recognition. Moreover, by configuring the AR device to determine the label for annotation from a corresponding video clip, there may be no need for, or reduced need for user intervention. Furthermore, in some aspects, the captured sensor data itself is used to automatically determine one of a plurality of subsequent data processing paths in the annotation engine. This can reduce the data labelling cost compared to traditional ad-hoc annotation processes where there needs to be dedicated trained workforces to perform the annotation tasks. Furthermore, annotation of the state data related to emotion/mental states is more accurate as no one understands one's actual feeling better than the user himself/herself, making relevant intelligent systems more personally effective and useful for users.

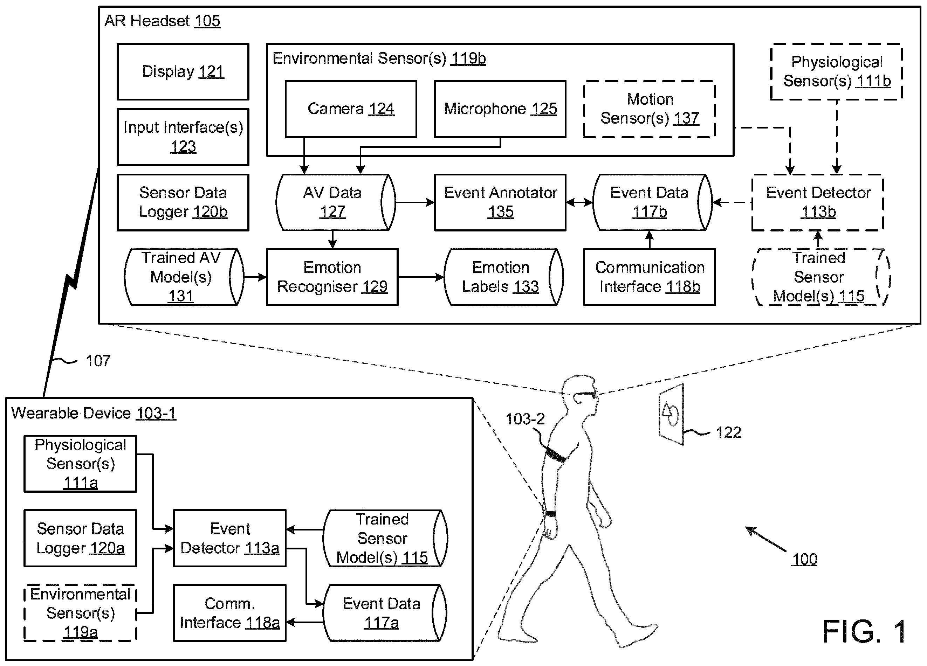

Figure 1 is a block flow diagram schematically illustrating an exemplary data processing system 100 for providing automatic annotation of captured physiological sensor data. As shown, in this example, the system 100 includes one or more wearable devices 103 in communication with an AR headset 105 via a data communication connection 107, such as a Bluetooth or Wi-Fi connection.

Each wearable device 103 includes one or more physiological sensors 111a to capture physiological signals, for example via a sensor data logger 120a. The sensor data logger 120a may record and store physiological signals at defined time intervals as captured sensor data in a memory of the wearable device 103. Each wearable device 103 also includes an event detector 113a to recognise potential events based on the physiological signals captured by the physiological sensors 111a. The event detector 113a processes the captured physiological sensor data using one or more trained sensor data processing models 115 to detect or predict potential events captured by the physiological sensor data. For example, the event detector 113a and trained sensor data models 115a may implement a detection algorithm to detect physiological patterns corresponding to learned emotional or un-neutral mental states. The detection algorithm can be trained based on predefined labelled physiological data collected in advance. The event detector 113a can also be trained to detect neutral mental or non-emotive states in a user's physiological signal using pre-collected data.

Each wearable device 103 stores event data 117a identifying the detected potential events, for transmission to the AR headset 105 via a communication interface 118a and the data connection 107. The wearable devices 103 may also include one or more environmental sensors 119-1, such as an accelerometer or gyroscope, as shown in dashed lines in Figure 1. The event detector 113 may be further configured to detect or predict potential events captured by the environmental sensor data, for example using a corresponding trained sensor data processing model 115.

As shown, the AR headset 105 has a display 121 to output images 122 for example as a virtual interface including rendered objects that is mixed with the user's actual view of the real world, and an input interface 123 to receive user input. In the present exemplary embodiment, the AR headset 105 includes one or more environmental sensors 119-2, such as a camera 124 and a microphone 125. A sensor data logger 120b may record and store data signals from the environmental sensors 119-2 at defined time intervals as time-stamped audio-visual (AV) data 127 in a memory of the AR headset 105, for subsequent processing by an emotion recogniser engine 129. The emotion recogniser engine 129 implements pre-trained application routines and/or algorithms to detect certain events in the surround environment of the user that might potentially trigger certain un-neutral mental states in the user. For example, emotion recogniser engine 129 may recognise user emotion based on input AV data 127 captured by the camera 123 and/or microphone 125. The emotion engine 129 processes the captured AV data 127 using one or more trained AV data processing models 131 to determine emotion labels 133 corresponding to the recognised user emotions. The AR headset 105 also includes an event annotator 135 to automatically, or semiautomatically with user confirmation, annotate time-stamped events in event data 117b, for example as received from the, or each, wearable device 103 via a communication interface 118b and the data connection 107. The event annotator 135 may be configured to automatically annotate new event data records in response to receiving data from the connected wearable devices 103.

The AR headset 105 may also include one or more physiological sensors 11 1b to capture respective physiological signals, and a respective event detector 113b to detect or predict potential events from the physiological sensor data captured by the AR headset 105, using the one or more trained sensor data processing models 115. The event detector 113b of the AR headset 105 may be further configured to detect or predict potential events captured by logging and analysing sensor data from one or more additional built-in environmental sensors 119a, such as an accelerometer or gyroscope motion sensor 137 as shown in dashed lines in Figure 1, in combination with the audio-visual signal to detect potentially triggering events from the surrounding environment.

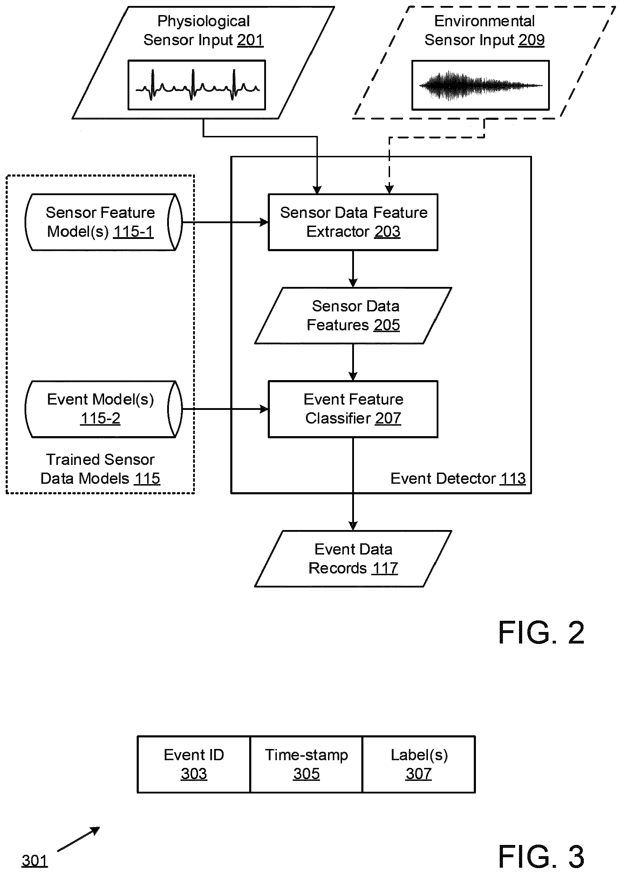

Figure 2 is a block data-flow diagram showing an exemplary implementation of an event detector engine 113. In this example, a physiological sensor input 201 is processed to generate one or more event data records 117. The event detector 113 may include a sensor data feature extractor 203 that receives and processes the physiological sensor input 201 using one or more trained sensor feature models 115-1 to extract one or more predefined features 205. The sensor data feature extractor 203 may use any known technique for converting the sensor input 201 to signal data based features that are suitable as input to a machine learned model or detection algorithm. For example, the sensor feature models 115-1 may be trained to extract one or more predefined statistical features (e.g. mean, standard deviation, min, max, etc.) of physiological information in the captured physiological sensor data 201, such as Blood Volume Pulse (BVP), Skin Temperature (ST), or Galvanic Skin Response (GSR), retrieved from the one or more wearable devices 103.

An event feature classifier 207 receives and processes the feature data 205 of the captured sensor input 201 using one or more trained event models 115-2, for example to classify the extracted features 205 and identify one or more events based on the classified features. The event feature classifier 207 also determines a time-stamp of each identified event based on the sensor input 201, such as a start time of an identified event corresponding to the time-stamp of a classified feature that triggers the recognised event. For example, the event feature classifier 207 may implement a machine-learned model or algorithm such as a neural network model that is trained to classify, detect and/or predict a user's mental state based on training data including the predefined statistical features of the physiological information and corresponding mental-state labels. Optionally, the event detector 113 may be further configured to receive and process environmental sensor input 209 for example using corresponding trained sensor data models 115 to identify one or more events based on the captured data from the corresponding environmental sensor(s) 119.

The event feature classifier 207 may also identify one or more labels associated with the identified event based on the trained event model(s) 115-2, each label defining for example a machine-recognised emotional state captured in the processed sensor data. Alternatively, an unlabelled event data record 301 may be generated by the event feature classifier 207 to be subsequently processed by the event annotator 135, for example where the trained event model(s) 115-2 cannot be used to classify a particular set of extracted features 205.

Detected event information and event-related data may be stored as a time-stamped event data 117 in a memory of the AR headset 105. Figure 3 schematically illustrates an exemplary record 301 of tracked object data 23. The event data record 301 may include an event identifier 303 and a time-stamp 305 of each identified event. The time-stamp 305 may be used by the event feature classifier 203 to store a start time of the identified event trigger based on the physiological sensor input 201 and/or environmental sensor input 209. The time-stamp 305 may also include an end time and/or duration of each event. The event data record 301 may also include one or more event label identifiers 307, for example used by the event feature classifier 207 to store the determined label associated with the identified event.

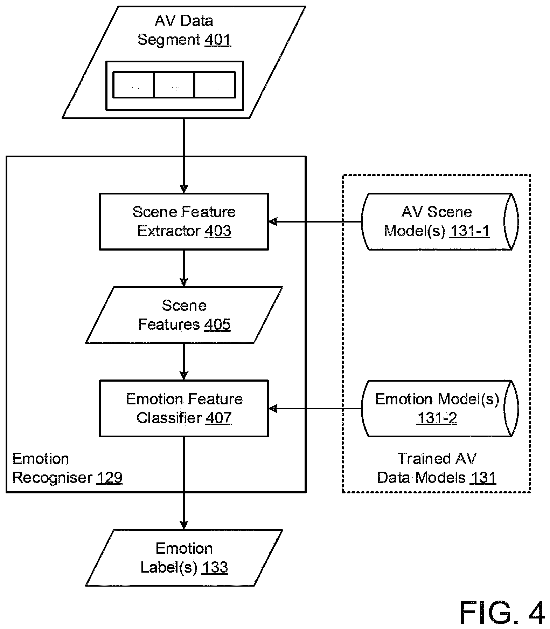

Figure 4 is a block data-flow diagram showing an exemplary implementation of an emotion recogniser engine 129. In this example, an input AV data segment 401 is processed to generate one or more recognised emotion labels 133. The AV data segment 401 may include one or more frames of video data captured by the camera 123 and/or audio data captured by the microphone 125. The emotion recogniser engine 129 includes a scene feature extractor 403 that receives and processes the input AV data segment 401 using one or more trained AV scene models 131-1 to extract one or more predefined scene-based features 405. The scene feature extractor 403 may use any known technique for converting the AV data input 401 to audio-visual data based features associated with the captured scene that are suitable as input to a machine learned model or detection algorithm. For example, the scene feature extractor 403 may implement image and/or audio data based object detection using a trained model, to output data identifying one or more scene-related objects detected in time-stamped frames of the input AV data 401. As an example, features such as frequency or pitch can be used for training neural network models to detect barking sounds of dogs. At the same time, visual features of video frames such as contour shape and closedness, combined with visual tracking across consecutive frames can be fed into deep learning models to recognize different postures, activities (e.g. running, jumping) or facial expressions of humans or animals in the video.

An emotion feature classifier 405 receives and processes the extracted scene-related features 405 using one or more trained emotion models 131-2, for example to classify the extracted features and identify one or more data labels 133 based on the classified features. The label data 133 may identify emotional state information associated with one or more sub-segments of the input AV data segment 401. The AV data models 131 can be pre-trained based on training data collected from different AV data sources, such as videos on social networks, with associated tags indicating relevant events and associated emotions in the videos. For example, the extracted scene-related features 405, such as recognized expressions, activities, and sounds in a scene, can be used to train a neural network model to output data representing a semantic understanding of the scene. For instance, the extracted scene-related features 405 may include data defining a scene where a dog is aggressively barking at someone or something in front of it, and the dog's owner is exhibiting traits of panic, thus corresponding to label data 133 identifying a scared emotional state of the user who is facing or witnessing the captured scene. The emotion data labels 133 may be stored in a memory of the AV headset 5 for retrieval and use by the event annotator engine 135, for example to annotate unlabelled event data records 117.

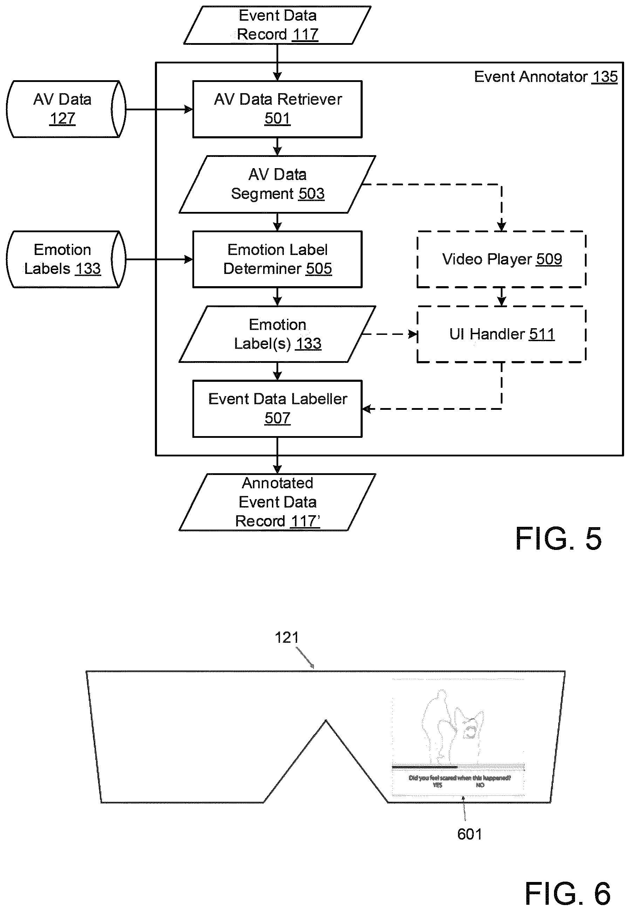

Figure 5 is a block data-flow diagram showing an exemplary implementation of an event annotator engine 135. In this example, an input event data record 117 is processed to generate a corresponding annotated event data record 117'. The input event data record 117 may be an unlabelled event generated by the event detector 113 and retrieved from memory. The event annotator 135 includes an AV data retriever 501 that identifies the time-stamp 305 of the input event data record 117 and retrieves the AV data segment 403 corresponding to the input time-stamped event, from the AV data store 127. An emotion label determiner 505 retrieves the corresponding emotion data label(s) 133 associated with the retrieved AV data segment, as determined and stored in memory by the emotion recogniser 129. An event data labeller 509 annotates the input time-stamped event with the retrieved emotion label(s) 133, for example by updating the label field 307 of the annotated event data record 117' with the retrieved label data 133 associated with the retrieved AV data segment 503.

In some aspects, the event annotator engine 135 may implement a user confirmation interaction to be performed by the AR headset 105. The retrieved AV data segment 503 may be output to the display 121, for example by a video player module 509. The retrieved emotion label(s) 133 may also be output to the display 121, for example as a textual prompt generated by a UI handler module 511. Figure 6 schematically illustrates an example of a virtual interface 601 output to the display 121 of the AR headset 5. The virtual interface 601 includes a prompt to the user for input confirmation to annotate a particular segment of captured physiological sensor data based on a corresponding labelled AV data segment. For example, the video segment may be accompanied by a question asking the user to confirm or assign the machine-predicted or recognised mental state corresponding to the event in the video. The UI handler module 511 may receive user input confirmation via the input interface(s) 123 before the event data labeller 507 proceeds to annotate the event data record 117'.

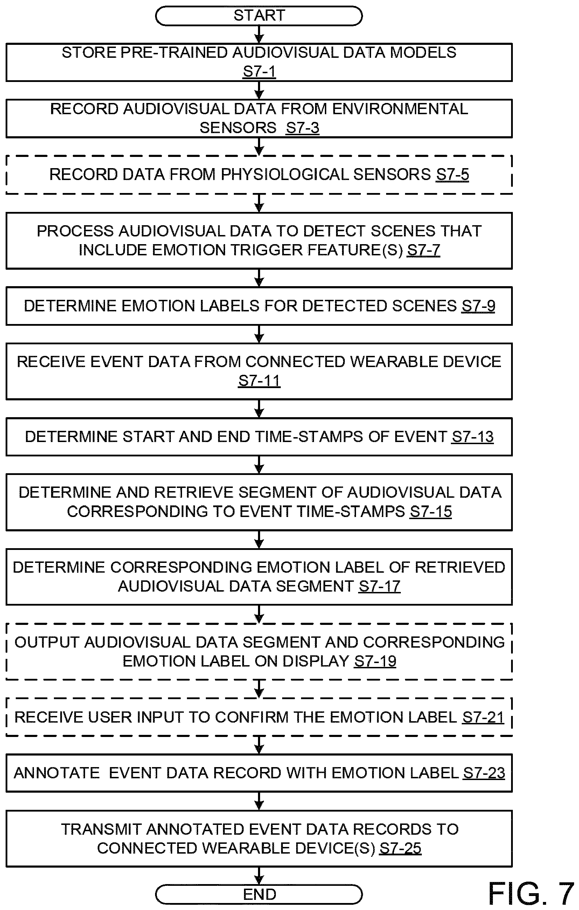

Figure 7 is a flow diagram illustrating an exemplary method of automatically annotating predicted events captured in physiological sensor data, performed by aspects of the AR headset 105. As shown, the process may begin at step S7-1 where the AR headset 105 stores trained audio-visual data models 131 in a memory. The audio-visual data models 131 may be pre-trained at a remote server and transmitted to the AR headset 105 via a data network. At step S7-3, the sensor data logger 120b begins to record audio-visual data captured by the environmental sensors 119b of the AR headset 105, such as the camera 124 and microphone 125. Optionally, the sensor data logger 120b may begin to record physiological data captured by the environmental sensors 119b of the AR headset 105, at step S7-5 shown in dashed lines.

At step S7-7, the emotion recogniser 129 processes the captured audio-visual data to detect scenes that include one or more emotion trigger features, and determines one or more associated emotion labels 133 for the detected scenes at step S7-9. For example, the emotion recogniser engine 129 may process an input AV data segment 401 to detect a dog barking at the user or a car crossing red light signal in front of the user, and classify the detected scene features with a learned prediction that the user might be scared by the corresponding real world aspects.

At step S7-11, the event annotator 135 receives event data 117 from a connected wearable device 103. The event data 117 may include one or more time-stamped event data records 301. At step S7-13, the event annotator 135 determines a start and end time-stamp of the, or each, received event, for example based on the time-stamp 305 of each data record 301. At step S7-15, the AV data retriever 501 of the event annotator 135 determines and retrieves a segment of audio-visual data 401 corresponding to the event start and end time-stamps. For example, the AV data retriever 501 may search the AV data 127 for a time-stamped AV data segment 401 that at least partially overlaps the event start and end time-stamps determined at step S7-13. At step S7-17, the emotion label determiner 505 of the event annotator 135 determines the corresponding emotion label(s) 133 of the AV data segment 401 retrieved at step S7-15. For example, the emotion label determiner 505 may retrieve from memory the emotion label(s) 133 associated with the retrieved AV data segment 401 as classified and stored by the emotion feature classifier 407. Alternatively, the event annotator 135 may use the emotion recogniser 129 to process the retrieved AV data segment 401 to determine one or more emotion labels 133 based on the trained AV data models 131.

In some aspects, the event annotator engine 135 may implement a user confirmation interaction to be performed by the AR headset 105. Accordingly, at step S7-19 shown in dashed lines, the event annotator engine 135 may generate and output a virtual interface 122 on the display 121, including playback of the retrieved AV data segment 503 in a region of the virtual interface 122 for example by the video player module 509. The corresponding emotion label(s) 133 determined at step S7-17 may also be output to the display 121, for example as a prompt for user input confirmation that the retrieved label 133 corresponds to the user's actual mental or emotional state as triggered by the scenes and events captured in the AV data segment 503 being played back on the display 121. At step S7-21 also shown in dashed lines, the event annotator 135 may receive user input via the input interface(s) 123 of the AR headset 105, for example to confirm that the emotion label 133 is accurate in response to the output prompt to the user. At step S7-23, the event data labeller 507 of the event annotator 135 proceeds to annotate the event data record 117' with the retrieved emotion label(s) 133, optionally in response to user input confirmation at step S7-21. At step S7-25, the event annotator 135 may transmit the annotated event data records 117' back to the respective connected wearable devices 103.

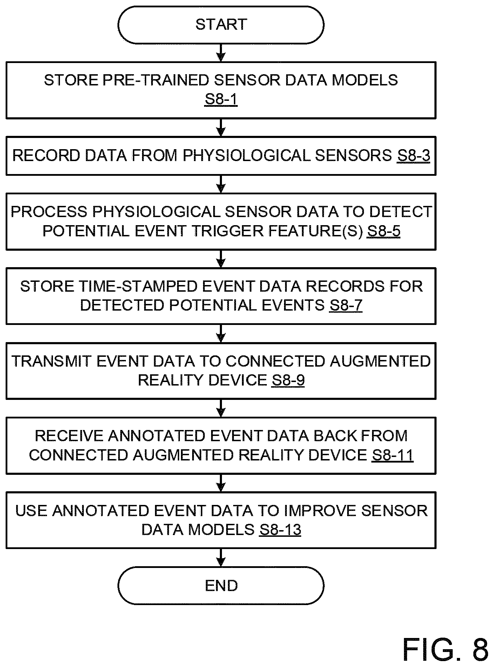

Figure 8 is a flow diagram illustrating an exemplary method of automatically improving the sensor data models 115 performed by aspects of the connected wearable devices 103. As shown, the process may begin at step S8-1 where the wearable device 103 stores trained sensor data models 115 in a memory. The sensor data models 115 may be pre-trained at a remote server and transmitted to the wearable device 103 via a data network. At step S8-3, the sensor data logger 120a of the wearable device 103 begins to record physiological data captured by the environmental sensors 119a. At step S8-5, the event detector 113a of the wearable device 103 processes the captured physiological sensor data to detect potential event trigger features, using one or more trained sensor data processing models 115 to recognise potential events captured by the physiological signals, such as physiological patterns corresponding to learned emotional or un-neutral mental states. The event detector 113a may also detect neutral mental or non-emotive states in the captured physiological sensor data. At step S8-7, the event detector 113a stores time-stamped event data records 117 for each detected potential event, in a memory of the wearable device 103. The event data records 117 may be unlabelled. At step S8-9, the wearable device 103 transmits the event data records 117 to the connected AR headset 105 via the communication interface 118a. At step S8-11, the wearable device 103 receives annotated event data records 117' back from the AR headset 105. The wearable device 103 may replace the stored event data records 117 with the corresponding received annotated event data records 117'. At step S8-13, the wearable device 103 uses the annotated event data records 117' to improve the trained sensor data model(s) 115.

The annotated event data 117' may be sent to a central server configured to process the received data to improve the original models used for classifying mental states. For example, in response to receiving the annotated event data 117', the server may first extract the predefined features of the received event as well as the corresponding mental-state label. The extracted features may be combined with current set of features associated with the extracted mental-state label stored by the server. The server may then perform processing to re-train the neural network model using updated training data including the new combined set of features, in order to be able to better recognise the extracted mental state. After the server finishes the re-training process, the new/updated recognition/classification model may be transmitted back to the wearable and AR devices. Additionally, when the server determines that the event data is annotated with a new (i.e. previously unused in the system) mental-state label, the server may also be configured to re-train an updated model or train a new model using the newly received annotated data, whereby the new or update model is able to recognize that new mental state. Subsequently received annotated event data associated with that new mental state may be used to yet further improve the model by re-training as discussed above.

In an alternative implementation, the system 100 may implement annotation of retrieved time-stamped event data based on a dynamic event detection process, whereby the event annotator 135 of the AR headset 105 may select one of a plurality of paths responsive to the processed AV data 127 and/or captured data from the one or more physiological sensors 111. Table 1 shows an example of three types of detection events that may be generated by the event detector 113 of the wearable device and/or AR headset 105 based on the captured sensor data, according to the present exemplary alternative implementation.

**Table 1**

| Signal Processing Outcome | Recognised State | Recorded Event |
|---|---|---|
| Pattern recognised in segment of physiological sensor data | Known label from trained model(s) | Detection Event Type I |
| Anomaly detected in physiological sensor data | Unrecognised | Detection Event Type II |
| Event detected in segment of environmental sensor data and/or audio-visual data | Known label from trained model(s) | Detection Event Type III |

As shown, the event detector 113a of the wearable device 103 may trigger and generate an event data record for a Type I detection event when the event feature classifier 207 detects the presence of one or more pre-trained patterns in the captured physiological sensor data 201 that correspond to an un-neutral mental or emotional state. The event feature classifier 207 may determine a classified label 307 of the detected un-neutral mental or emotional state based on the trained sensor model(s) 115. On the other hand, the event detector 113a of the wearable device 103 may trigger and generate an event data record for a Type II detection event when the event feature classifier 207 detects that the captured physiological data is different from a defined neutral mental or emotional state but the pattern of extracted sensor data features 205 cannot be classified based on the trained event model(s) 115-2. For example, the event feature classifier 207 may be configured to determine that an anomaly is present in the physiological signal and to label the Type II detection event as unrecognised. The recorded event data record(s) 117a, including the identified label(s) 307 and the corresponding timestamps 305 are then transmitted to the AR headset 105, as discussed in the embodiment above.

The event detector 113b of the AR headset 105 may trigger and generate an event data record for a Type III detection event when the event feature classifier 207 detects the presence of one or more pre-trained patterns in the captured physiological sensor data 201 and/or environmental sensor data 209 that correspond to potentially triggering events from the surrounding environment. For example, when the user needs to move quickly to avoid an object or person in the real world, such sudden movement changes would be captured by the built-in motion sensors 137 and detected by the event detector 113b. The event feature classifier 207 may determine a classified label 307 of the detected triggering event based on the trained sensor model(s) 115. The recorded event data record(s) 117b, including the identified label(s) 307 and the corresponding timestamps 305 are then logged in a memory of the AR headset 105.

When the event detector 113b of the AR headset 105 subsequently detects that either the captured physiological signals 201 of the user is back to a neutral mental or emotional state, or the environmental sensor data 209 is no longer capturing any triggering events in the surrounding environment, the AR headset 105 may proceed to initiate the event data annotation process as discussed in the embodiment above.

In some implementations, the AR headset 105 may display a virtual interface 601 in front of the user, for example to prompt for user input in the process to automatically label a captured physiological signal. As physiological signals are typically not intuitive to normal users, instead of showing the values of the physiological signals, the AR headset 105 may display the captured video segment corresponding to the chunk of physiological signal where un-neutral mental or classified emotional states might have been triggered to the user. Audio-visual cues will be more intuitive, memorable and effective to remind the user about certain events as well as their associated mental or emotional state.

In an exemplary implementation, the event annotator 135 of the AR headset 105 may be configured to determine and output a different UI prompt based on the selected path, i.e. depending on the type of detection event. For example, if a detection event of Type I was triggered, the user may be prompted to perform a binary confirmation of the recognised emotion label 133.

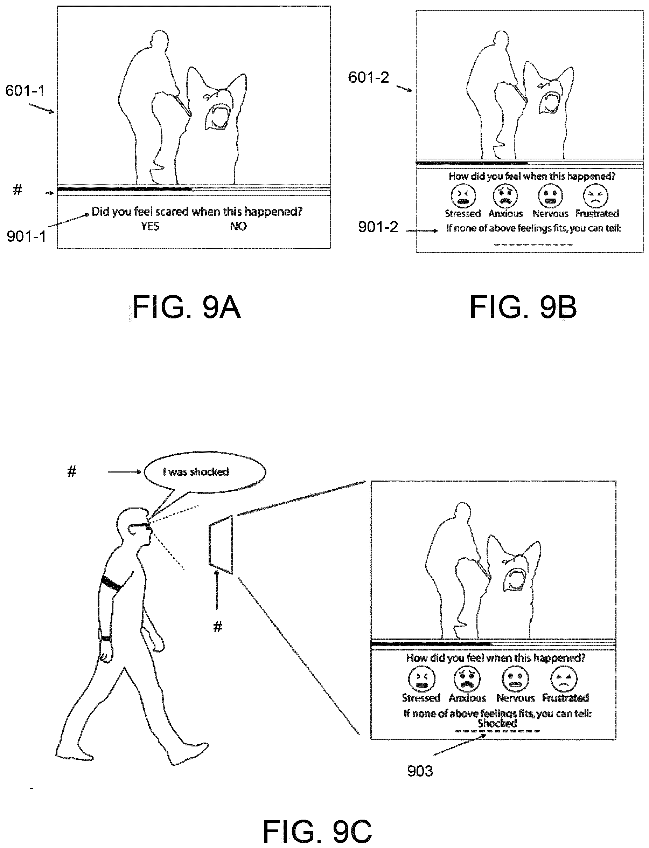

Figure 9A shows an exemplary AR interface 601-1 where the user can binarily confirm a mental or emotional state detected by the system 100 to be potentially associated to the displayed video segment and its corresponding physiological classified label 133. For example, the displayed interface 601-1 can include an accompanying text prompt 901-1 asking "Did you feel scared when this happened? Yes / No" and the user can select between "Yes" and "No" to confirm whether he felt scared by the event in the video, for example via suitable input means.

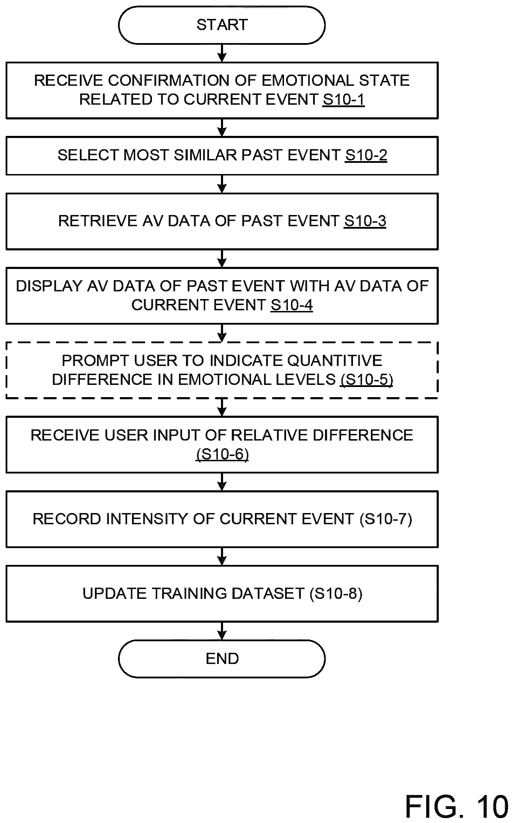

The event annotator 135 may allow the user to further specify a fine-grained, quantitative level of the emotion that the user was perceiving in response to a current event, not only limited in a binary manner (Yes or No). For an identified emotion, the user can also indicate its intensity that he/she was feeling. As humans are better at indicating the relative difference between multiple levels of an emotion than specifying an absolute level of the emotion, the event annotator 135 allows the user to quantify the level of the emotional state in connection with a candidate event, relative to a similar event he/she experienced in the past. The following method, as shown in the flowchart of Figure 10, allows a user to specify the quantitative level of emotion being perceived.

First, in response to the user confirming the label of an existing emotional state in response to a current or candidate event via the interface (S10-1), the system retrieves from data representing past or other events the one where the user has experienced the most similar emotion (S10-2). The event with the physiological signal data closest to the physiological signal data currently under examination may be considered to be the most similar to the emotional state the user has recently confirmed. An exemplary approach to determine the similarity/closeness between two sets of physiological signal data is to compute the mean and the standard deviation value of the differences between them. The more similar two sets of physiological signal data are to each other, the smaller the mean and the deviation value of the differences between them.

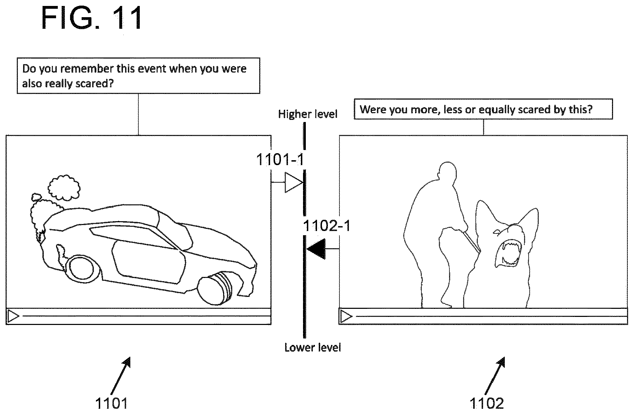

Once the closest physiological signal data with the same emotion label is identified, the corresponding audio-visual data is then retrieved (S10-3) and displayed (1101) together with the audio-visual data (1102) of the current event (S10-4), as shown for example in Figure 11. The event annotator 135 may provide a prompt through the AR/MR headset interface (e.g. display 121) to ask the user to indicate the relative quantitative difference in the emotion levels the user perceived in the two events represented by the two audio-visual segments shown on the display (S10-5). Then, the event annotator 135 may receive input from the user (e.g., using hand gestures, voice or possibly gaze to interact with the interface) to visually indicate if the events in the two audio-visual segments are equivalent or different (S10-6). In one embodiment of interaction, the user can interact with the AR/MR headset interface to adjust the positions of graphical elements (1101-1, 1102-1) corresponding to the two events on a scale to visually indicate the perceived difference in the emotion. In this embodiment, the initial position of the past event on the scale can be determined based on the level of emotion assigned to the corresponding physiological signals. The position of the current event on the scale can be initialized based on the relative difference between the two mean values of the two corresponding physiological signals multiplied by a predefined constant value. Thus, if the two physiological signals are mostly similar to each other, the positions of the graphical elements corresponding to the two events on the scale are close to each other. If the mean values of the physiological signals associated to the current event and the past event are different, the graphical elements corresponding to the two videos are spatially separated.

In this exemplary embodiment, the user may provide input (e.g., using hand gestures, voice or gaze) to change the position of the graphical element (1102-1) corresponding the current event to indicate how much his/her corresponding emotion was stronger, weaker or equivalent to the emotion perceived in the past event (e.g., to the left to indicate a weaker emotion or to the right to indicate a stronger emotion, or to the position of the past event to indicate a similar level of emotion). For example, using hand gestures, the user can perform a grabbing gesture with his/her fingers and drag the graphical element along the scale. In another exemplary embodiment using voice interaction, the user can verbally command to the event annotator 135 as "It was weaker/stronger" or "It might be less, to the left/more, to the right" until the graphical element reached the desired position on the scale. The position of the graphical element (1102-1) corresponding to the current event on the scale in comparison to the past event will be converted to the intensity of the emotion perceived by the user, which will then be recorded (S 10-7) together with the label of the emotional state and the corresponding physiological and input into the training dataset to improve the emotional state recognition model (S10-8).

On the other hand, if a detection event of Type II was triggered (for example with an unrecognized state label 307), the event annotator 135 of the AR headset 105 may determine if another detection event of Type III was recorded at the same time, based on comparison of the logged timestamps 305. If a detection event of type III was detected at the same time as the detection event of Type II, the user may be prompted via the virtual interface 601 to confirm if the event in the video triggered the mental or emotional state as classified based on the detection event of Type III. For example, if the physiological signals detect the presence of an unrecognised or undefined un-neutral mental/emotional state of the user (i.e. triggered a detection event of Type II), and the system 100 detected from the audio-visual signal the presence of a dog aggressively barking at the user, the AR headset 105 may display the corresponding video segment accompanied with a textual prompt such as "Did this event scare you? Yes / No". Similar to the previous example, the user can then choose between "Yes" and "No" via suitable input means, which will be used to proceed with automatic labelling of the corresponding physiological data as discussed in the embodiment above.

In yet a further alternative implementation, if it is determined that another detection event of Type III was not recorded at the same time as the detection event of Type II, the event annotator 135 may rank the set of all pre-defined un-neutral mental/emotional states from the trained emotion model(s) 131-2, to identify a subset of mental/emotional states that have scene feature patterns closest or most similar to the patterns of scene features 403 extracted from the captured physiological signals. Figure 9B shows an exemplary AR interface 601-2 including a displayed subset of mental/emotional states determined to be most similar to the physiological signal of the user, together with the corresponding video segment, and a prompt 901-2 for the user to select one of the mental/emotional states in the subset list to be used as the associated label 307. The user can confirm, via suitable input means, a selected one of the states from the displayed list of mental/emotional states.

The AR headset 105 may also be configured to allow the user to assign a new mental/emotional state label in case none of listed states match the user's actual perceived mental state at the time of the video segment. Figure 9C shows another exemplary AR interface 601-3 and schematically illustrates how the user can use speech input to label the data with a new state label 133. As shown, the event annotator 135 of the AR headset 105 may be configured to allow the user to provide a free form answer through speech input via the microphone 125. For example, the user can say "I was shocked", where "Shocked" was not in the output list of determined closest matching states. The AR headset 105 may process the speech input, using any known technique, to extract the word(s) describing the user's mental state and use the extracted word(s) as a new classified label 133 of the event data record 117.

In this way, the emotion/mental state label confirmed or assigned by the user may be used to primarily label the corresponding physiological signals in order to improve the detection accuracy of the user's mental state based on physiological data in the future. Additionally, the label can also be used to annotate the corresponding audio-visual data to further improve the event recognition algorithm for AV data.

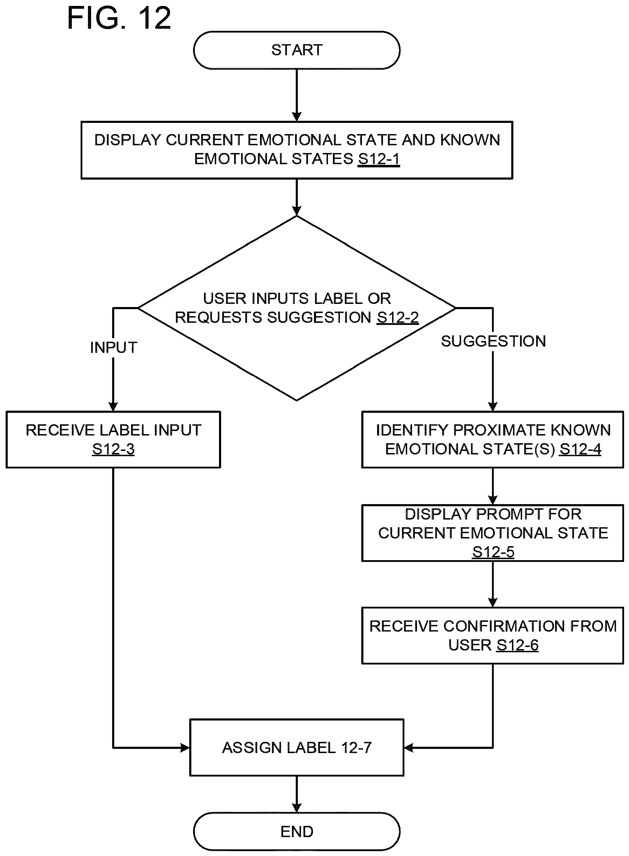

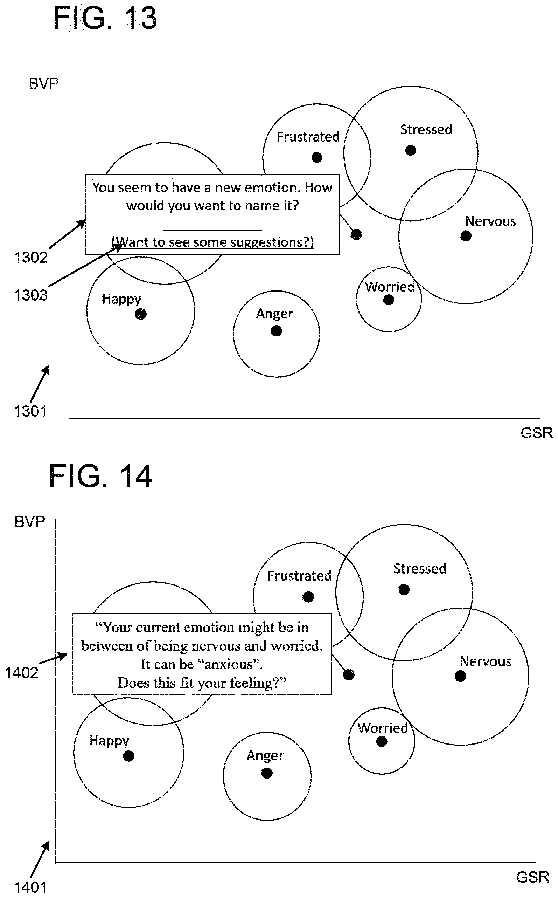

There may be also a case where the user does not know how to name the label of the new emotion. In this case, the event annotator 135 may provide the user with a method to label the new emotion based on a spectrum of emotion constructed from the emotional states known by the current models and their corresponding physiological range of data. In an exemplary embodiment as shown in Figs. 12 and 13, the event annotator 135 may display (step S12-1) the existing emotion labels on a 2D chart as shown in Fig. 13, where the two axes of the coordinate system may be characterized by two types of physiological data such as BVP or GSR. The existing labels of mental state are plotted on this coordinate system based on their ranges of those two types of physiological data. The physiological signals currently under examination are also plotted on this chart. The spatial proximities of the visualization of these physiological signals to existing emotion labels can assist the user in answering the event annotator 135 via the interface with a suitable label for the emotional state to be annotated, as shown in Fig. 13.

In the user interface display 1301 of Figure 13, the position of each emotional state on the chart (represented by a dot or other mark) is determined by the mean values of all samples in the category along the two axes and the size (represented by a circle or other area around the dot or other mark) represents the average standard deviation value. The position of the new emotional state is also plotted on the chart based on the mean values of its physiological signals. The user can input the name of the emotion by themselves or select to see suggestions of the event annotator 135 (step S12-2).

Furthermore, the event annotator 135 can intelligently provide a hint to the user about the name of the emotion. To do this, for example, the user can select the "Want to see some suggestions" link 1303 on the user interface display 1301. To achieve this intelligent support, the event annotator 135 calculates (step S12-4) the proximities of the emotional state currently under examination to existing emotions on the chart to automatically suggest the user with a hint to name the new emotion. For example, the event annotator 135 may use the distances between their mean values as proximity scores to identify a predefined number of emotional states closest to the current emotional state. If the proximity scores of these closest emotions are smaller than a predefined threshold, they will be considered proximate emotions to the new one. Otherwise, if the proximity score between an existing emotion to a new one is smaller than the size of the existing emotion, it is still considered to be proximate to the new one. Based on the most proximate emotions identified, the event annotator 135 provides the user with a naming prompt (Step S12-5). For example, as shown in Fig. 14, if the event annotator 135 identifies that the existing emotions most proximate to the current one are "nervous" and "worried", it automatically generates a prompt such as "Your current emotion might be in between of being nervous and worried".

Moreover, the event annotator 135 may also automatically generate the name of the new emotion based on proximate ones using a pre-trained AI system (for example using a large language model). For example, the system may create a prompt from the identified proximate emotions (e.g., "What is the emotion between nervous and worried") and send this prompt as input to the pre-trained AI system. The AI system may respond with a suggesting sentence including a name for the new emotion (for example, "The emotion between nervous and worried is anxious") to the system. The event annotator 135 processes this sentence to extract the suggested emotion name (here: anxious) and creates a prompt on the AR/MR interface to hint the user as follows: "Your current emotion might be in between of being nervous and worried. It can be "anxious". Does this fit your feeling?" (see Figure 14). Then the user just needs to confirm (step S12-6) the suggestion if he/she finds it suitable through hand gestures, voice or gaze interaction.

The event annotator 135 then assigns (step S12-7) the label, as determined in any of the processes described above, to the current emotional state. As the set of physiological signals under examination is the first instance in the newly defined emotional state, the event annotator 135 may assign a default value of intensity for this instance. This is the baseline value which will be used to calibrate the intensity of other physiological signals to be assigned with the same emotional state label in the future.

### Example Computer System Implementation

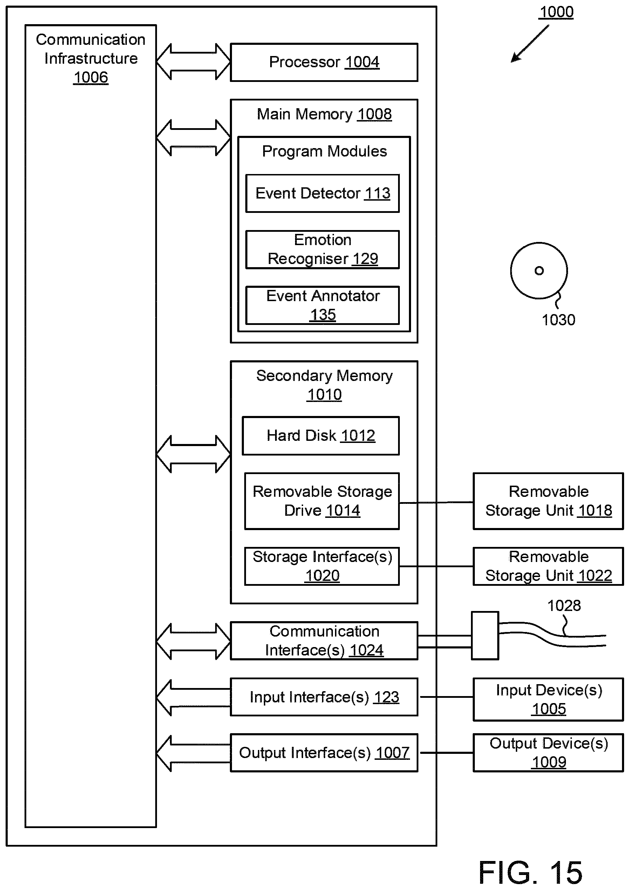

Figure 15 illustrates an example computer system 1000 in which the present invention, or portions thereof, can be implemented as computer-readable code to program processing components of the computer system 1000. Various embodiments of the invention are described in terms of this example computer system 1000. For example, the AR headset 105 and wearable devices 103 of Figure 1 can each be implemented in system 1000. The methods illustrated by the flowcharts of Figures 7, 8, 10 and 12 can also be implemented in system 1000. After reading this description, it will become apparent to a person skilled in the relevant art how to implement the invention using other computer systems and/or computer architectures.

Computer system 1000 includes one or more processors, such as processor 1004. Processor 1004 can be a special purpose or a general-purpose processor. Processor 1004 is connected to a communication infrastructure 1006 (for example, a bus, or network). Computer system 1000 also includes a user input interface 1003 connected to one or more input device(s) 1005 and a display interface 1007 connected to one or more output devices 1009, such as display 121, which may be integrated input and display components. Input devices 1005 may include, for example, a connected device such as a mouse or touchpad, a keyboard, a touchscreen such as a resistive or capacitive touchscreen, etc.

Computer system 1000 also includes a main memory 1008, preferably random access memory (RAM), and may also include a secondary memory 1010. Secondary memory 1010 may include, for example, a hard disk drive 1012, a removable storage drive 1014, flash memory, a memory stick, and/or any similar non-volatile storage mechanism. Removable storage drive 1014 may comprise a floppy disk drive, a magnetic tape drive, an optical disk drive, a flash memory, or the like. The removable storage drive 1014 reads from and/or writes to a removable storage unit 1018 in a well-known manner. Removable storage unit 1018 may comprise a floppy disk, magnetic tape, optical disk, etc. which is read by and written to by removable storage drive 1014. As will be appreciated by persons skilled in the relevant art(s), removable storage unit 1018 includes a non-transitory computer usable storage medium having stored therein computer software and/or data.

In alternative implementations, secondary memory 1010 may include other similar means for allowing computer programs or other instructions to be loaded into computer system 1000. Such means may include, for example, a removable storage unit 1022 and an interface 1020. Examples of such means may include a program cartridge and cartridge interface (such as that found in video game devices), a removable memory chip (such as an EPROM, or PROM) and associated socket, and other removable storage units 1022 and interfaces 1020 which allow software and data to be transferred from the removable storage unit 1022 to computer system 1000.

Computer system 1000 may also include a communications interface 1024 implemented for example at the operating system level to allow data to be transferred between computer system 1000 and external devices, for example as signals 1028 over a communication channel 1026. Communications interface 1024 may include a modem, a network interface (such as an Ethernet card), a communications port, a PCMCIA slot and card, or the like.

Various aspects of the present invention, such as the event detector 113, emotion recogniser 129 and event annotator 135 program modules, can be implemented by software and/or firmware (also called computer programs, instructions or computer control logic) to program programmable hardware, or hardware including special-purpose hardwired circuits such as application-specific integrated circuits (ASICs), programmable logic devices (PLDs), field-programmable gate arrays (FPGAs), etc. of the computer system 1000, or a combination thereof. Computer programs for use in implementing the techniques introduced here may be stored on a machine-readable storage medium and may be executed by one or more general-purpose or special-purpose programmable microprocessors. The terms "computer program medium", "non-transitory computer readable medium" and "computer usable medium" introduced herein can generally refer to media such as removable storage unit 1018, removable storage unit 1022, and a hard disk installed in hard disk drive 1012. Computer program medium, computer readable storage medium, and computer usable medium can also refer to memories, such as main memory 1008 and secondary memory 1010, which can be memory semiconductors (e.g. DRAMs, etc.). These computer program products are means for providing software to computer system 1000.

Computer programs are stored in main memory 1008 and/or secondary memory 1010. Computer programs may also be received via communications interface 1024. Such computer programs, when executed, enable computer system 1000 to implement the present invention as described herein. In particular, the computer programs, when executed, enable processor 1004 to implement the processes of embodiments of the present invention as described above. Accordingly, such computer programs represent controllers of the computer system 1000. Where the invention is implemented using software, the software may be stored in a computer program product and loaded into computer system 1000 using removable storage drive 1014, interface 1020, hard drive 1012, or communications interface 1024.

### Alternative Embodiments

The descriptions of the various embodiments have been presented for purposes of illustration, but are not intended to be exhaustive or limited to the embodiments disclosed. Many modifications and variations will be apparent to those of ordinary skill in the art without departing from the scope and spirit of the described embodiments.

Aspects of the present embodiments may be embodied as a system, method or computer program product. Accordingly, aspects of the present disclosure may take the form of an entirely hardware embodiment, an entirely software embodiment (including firmware, resident software, micro-code, etc.) or an embodiment combining software and hardware aspects that may all generally be referred to herein as an "engine", a "module," a "system," or a "computer." In addition, any hardware and/or software technique, process, function, component, engine, module, or system described in the present disclosure may be implemented as a circuit or set of circuits. Furthermore, aspects of the present disclosure may take the form of a computer program product embodied in one or more computer readable medium(s) having computer readable program code embodied thereon.

Aspects of the present disclosure are described above with reference to flowchart and/or block diagrams of methods, apparatus (systems) and computer program products according to embodiments of the disclosure. It will be understood that each block of the flowchart and/or block diagrams, and combinations of blocks in the flowchart and/or block diagrams, can be implemented by computer program instructions. These computer program instructions may be provided to a processor of a general purpose computer, special purpose computer, or other programmable data processing apparatus to produce a machine. The instructions, when executed via the processor of the computer or other programmable data processing apparatus, enable the implementation of the functions/acts specified in the flowchart and/or block diagram block or blocks. Such processors may be, without limitation, general purpose processors, special-purpose processors, application-specific processors, or field-programmable gate arrays.

The flowchart and block diagrams in the figures illustrate the architecture, functionality, and operation of possible implementations of systems, methods and computer program products according to various embodiments of the present disclosure. In this regard, each block in the flowchart or block diagrams may represent a module, segment, or portion of code, which comprises one or more executable instructions for implementing the specified logical function(s). It should also be noted that, in some alternative implementations, the functions noted in the blocks of the flowchart diagrams may occur out of the order noted in the figures. For example, two blocks shown in succession may, in fact, be executed substantially concurrently, or the blocks may sometimes be executed in the reverse order, depending upon the functionality involved. It will also be noted that each block of the flowchart diagrams, and combinations of blocks in the flowchart diagrams, can be implemented by special purpose hardware-based systems that perform the specified functions or acts, or combinations of special purpose hardware and computer instructions.

While the preceding is directed to embodiments of the present disclosure, other and further embodiments of the disclosure may be devised without departing from the basic scope thereof, and the scope thereof is determined by the claims that follow.

## Claims

1. An augmented or mixed reality apparatus (105) comprising:
a display (121);
one or more environmental sensors (119b) including a camera (124);
a memory (1008;1010) to store video data (127) from the camera, and event data (117b) identifying at least one events (301) detected in data from one or more physiological sensors (111); and
one or more processors (1004) configured to:
process the video data to detect one or more segments of the video data corresponding to the at least one identified events (301) and to determine label data (133) comprising emotion information associated with the at least one identified event;
retrieve one or more segments of the video data corresponding to a selected one of said at least one identified event;
display to a user the retrieved one or more segments of the video data corresponding to the selected event;
prompt the user to input a quantitative level of emotion relating to the selected event; and
include said quantitative level in the label data (133) corresponding to the selected event.

2. The apparatus of claim 1, wherein the retrieved one or more segments of video data comprises a first one or more segments of video data, the one or more processors being further configured to:
retrieve (S10-3) a second one or more segments of video data having associated second label data (133), the second segment of stored video data being selected (S10-2) for retrieval based on similarity between the emotion information in the label data associated with the first and second segments of video data;
output (S10-4) on said display (121) said first and second segments of video data; and
receive (S10-6) said user input of a quantitative level of emotion associated with the first segment of video data in comparison with the second segment of video data.

3. An augmented or mixed reality apparatus (105) comprising:
a display (121);
one or more environmental sensors (119b) including a camera (124);
a memory (1008;1010) to store video data (127) from the camera, and event data (117b) identifying one or more events (301) detected in data from one or more physiological sensors (111); and
one or more processors (1004) configured to:
retrieve from the video data (127) one or more video segments corresponding to the at least one identified event;
display the retrieved video segment(s) to a user;
compare physiological sensor data corresponding to the retrieved segment(s) of video data with physiological sensor data corresponding to one or more known emotional states;
determine, based on said comparison, one or more candidate emotional states corresponding to the retrieved segment(s) of video data;
output said one or more candidate emotional states to the user; and
in response to confirmation of an emotional state by the user, annotate the event data corresponding to the retrieved segment(s) of video data with label data indicating the emotional state confirmed by the user.

4. The apparatus of claim 3, wherein the one or more candidate emotional states are selected from the known emotional states according to similarity between physiological sensor data of known emotional states and the physiological sensor data corresponding to the retrieved segment(s) of video data.

5. The apparatus of claim 4, wherein the one or more candidate emotional states are determined by selecting a plurality of known emotional states having similar physiological sensor data to that corresponding to the retrieved segment(s) of video data, providing the selected known emotional states as input to a pre-trained AI system, and obtaining in response from the AI system the one or more candidate emotional states.

6. The apparatus of any preceding claim, wherein the event data is received from a connected wearable device (103) and stored in the memory.

7. The apparatus of claim 6, wherein the one or more processors are further configured to transmit annotated event record back to the connected wearable device.

8. The apparatus of any preceding claim, wherein the corresponding one or more segments of the video data are determined based on time stamp data in the event data.

9. The apparatus of any preceding claim, wherein the one or more processors are configured to detect the one or more segments of the video data corresponding to the at least one identified event using a trained model.

10. A wearable device comprising:
one or more physiological sensors (111a); and
one or more processors (1004) configured to:
process signal data from the one or more physiological sensors using a trained data model (115) to detect an event occurrence in the signal data that cannot be classified according to pre-trained labels of the trained model;
generate an unlabelled time-stamped event record based on the detected event occurrence;
transmit the unlabelled time-stamped event record to an augmented or mixed reality apparatus (105) as set out in any one of claims 1 to 8;
receive a labelled version of the time-stamped event record from the augmented or mixed reality apparatus; and
use the received label to improve the trained data model.

11. A computer-implemented method of operation of the apparatus of any one of claims 1-2 or claims 6-9 when dependent thereon, comprising:
storing the video data (127) captured by a camera;
storing event data (117b) identifying one or more events (301) detected in data from one or more physiological sensors (111);
processing the video data to detect one or more segments of the video data corresponding to the at least one identified event and to determine label data (133) comprising emotion information associated with the at least one identified event;
displaying to a user the retrieved one or more segments of the video data corresponding to the selected event;
prompting the user to input a quantitative level of emotion relating to the associated event; and
including said quantitative level in the label data (133) corresponding to the selected event.

12. A computer-implemented method of operation of the apparatus of any one of claims 3-5 or claims 6-9 when dependent thereon, comprising:
storing the video data (127) captured by the camera;
storing event data (117b) identifying one or more events (301) detected in data from one or more physiological sensors (111);
retrieving, from the video data, one or more video segments corresponding to the at least one identified event;
displaying the retrieved video segment(s) to a user;
comparing physiological sensor data corresponding to the retrieved segment of video data with physiological sensor data corresponding to one or more known emotional states;
determining, based on said comparison, one or more candidate emotional states corresponding to the retrieved segment(s) of video data;
outputting said one or more candidate emotional states to the user; and
in response to confirmation of an emotional state by the user, annotating the event data corresponding to the retrieved segment(s) of video data with the emotional state confirmed by the user.

13. A computer-readable storage medium comprising instructions which, when executed by a computer, cause the computer to carry out the method of claim 11 or 12.
